(19)

Europäisches Patentamt
European Patent Office
Office européen des brevets

(11) EP 2 202 539 A1

(12) **EUROPEAN PATENT APPLICATION**
published in accordance with Art. 153(4) EPC

(43) Date of publication:
**30.06.2010 Bulletin 2010/26**

(51) Int Cl.:
**G01T 1/161** (2006.01)          **A61B 6/03** (2006.01)
**G06T 1/00** (2006.01)          **G06T 5/00** (2006.01)
**G06T 5/20** (2006.01)

(21) Application number: **08790395.1**

(22) Date of filing: **06.08.2008**

(86) International application number:
**PCT/JP2008/002131**

(87) International publication number:
**WO 2009/050837 (23.04.2009 Gazette 2009/17)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **15.10.2007 JP 2007267859**

(71) Applicant: **Nihon Medi-Physics Co., Ltd. Tokyo 136-0075 (JP)**

(72) Inventors:
• **MAEDA, Hisato**
  **Toyoake-shi**
  **Aichi 470-1192 (JP)**
• **YAMAKI, Noriyasu**
  **Nishinomiya-shi**
  **Hyogo 662-0918 (JP)**

(74) Representative: **Wibbelmann, Jobst**
**Wuesthoff & Wuesthoff**
**Patent- und Rechtsanwälte**
**Schweigerstrasse 2**
**81541 München (DE)**

(54) **IMAGE PROCESSING PROGRAM, IMAGE PROCESSING METHOD AND IMAGE PROCESSOR**

(57) An image processing apparatus (1) comprises: a cutoff frequency table (26) storing information on a relation between a parameter related to a radial intensity distribution of two-dimensional frequency components of a projection image and a Butterworth filter cutoff frequency to be applied to the projection image concerned; a projection image data input unit (10) for inputting projection image data; an FFT unit (20) for applying a two-dimensional Fourier transform to the projection image to obtain a spatial frequency of the projection image; a radial intensity distribution calculation unit (22) for calculating a radial intensity distribution of frequency components of the projection image; a cutoff frequency setting unit (24) for setting a cutoff frequency corresponding to a parameter related to the radial intensity distribution determined for the projection image, with reference to the cutoff frequency table; and a Butterworth filter (12) for filtering the projection image data with the set cutoff frequency. Consequently, an optimum cutoff frequency can be set automatically to perform filtering.

【FIG.1】

EP 2 202 539 A1

**Description**

RELATED APPLICATIONS

This application claims the benefit of Japanese Patent Application No. 2007-267859 filed on October 15, 2007 in Japan, the contents of which are incorporated herein by reference.

TECHNICAL FIELD

The present invention relates to a method of setting a condition of a filter used for image processing of a nuclear medicine image, and to an image processing apparatus and program for carrying out the method. In particular, the invention relates to a method of determining a cutoff frequency of a Butterworth filter used for image processing of a nuclear medicine image, and to an image processing apparatus and program for carrying out the method. Nuclear medicine images to which the invention is applied include an image provided by positron emission tomography (PET) and an image provided by single photon emission computed tomography (SPECT).

BACKGROUND ART

Nuclear medicine images, typified by a PET image and a SPECT image, are effective for diagnosis of heart failure, cancer, and other various diseases. At the present time, diagnosis using a nuclear medicine image is made in 1200 facilities or more throughout Japan (Working Group for Investigation and Research on Nuclear Medicine Image Quantification and Standardization, the Japanese Society of Nuclear Medicine Technology, Questionnaire Report for Practical Conditions of Nuclear Medicine Examination and Standardization of Image Acquisition, Processing, Display and Output, the Japanese Journal of Nuclear Medicine Technology, Vol. 24, No. 2 (2004), 95-118).
In a nuclear medicine image examination, a drug labeled with a specific radioisotope (hereinafter referred to as a "radiopharmaceutical") is given to a subject, and gamma rays emitted directly or indirectly from the drug are detected by means of a dedicated camera. A plurality of image data obtained from the results of detecting gamma rays at different angles (hereinafter referred to as "projection data") are reconstructed to obtain a nuclear medicine tomogram. Here, acquired image data includes noise caused by statistical fluctuations. In general, creating a reconstruction image without removing noise from acquired image data cannot provide an image that can be used in diagnosis. In order to obtain a good image that can be used in diagnosis, it is required to remove noise included in image data, prior to an image reconstruction process.
As methods of removing noise included in image data, there are known a filtering process using a Butterworth filter, a Gaussian filter, or the like, a smoothing process, and the like. The most relied-upon and clinically widely-used method of these methods is a method in which image data is filtered with a Butterworth filter. In this method, image data is multiplied by a filter function that is defined by an order and a cutoff frequency.

DISCLOSURE OF THE INVENTION

Problems to be solved by the invention

Removing noise using a Butterworth filter requires that an order and a cutoff frequency be set. Of these parameters, a cutoff frequency is known to greatly affect image quality of a reconstruction image. Butterworth filtering without using an appropriate cutoff frequency may cause an incorrect diagnosis result.
Conventionally, a cutoff frequency of a Butterworth filter would be set based on the experience of an operator. That is, image processing would be performed a plurality of times with a cutoff frequency being varied, and an operator would choose from the plurality of obtained images an image that the operator considers to be optimum. Then, the operator would set the cutoff frequency used for obtaining the chosen image as an optimum value. However, this method has a problem that image quality of a nuclear medicine image to be obtained largely depends on the experience and skill of an operator. For image processing, a method is desired in which a cutoff frequency can be set uniquely, but such a method is hitherto unknown.
The above-mentioned document describes a result of a questionnaire addressed to all nuclear medicine treatment facilities that have a gamma camera. According to the questionnaire result, the most popular answer to "questions in a nuclear medicine examination and items that should be explained by equipment makers" is the setting of a cutoff frequency of a Butterworth filter.
A purpose of the invention made in view of the above-mentioned background is to provide an image processing apparatus, an image processing method, and a program that can perform filtering with an optimum cutoff frequency being set automatically.

Means for solving the problems

The present inventors have made intensive study addressing the technical issue of automatically setting an optimum cutoff frequency of a Butterworth filter and, as a result, have found that there is a correlation between a parameter related to a radial intensity distribution of a spatial frequency of a nuclear medicine image and an optimum cutoff frequency. Based on this finding, the inventors have completed the invention in which a Butterworth filter is set with an optimum cutoff frequency.

An image processing program of the invention is a program for performing image processing on nuclear medicine image data, and causes a computer to execute the steps of: inputting nuclear medicine image data; applying a two-dimensional Fourier transform to the inputted nuclear medicine image to obtain a spatial frequency of the nuclear medicine image; calculating a radial intensity distribution of the spatial frequency of the nuclear medicine image; determining a cutoff frequency corresponding to a parameter related to the radial intensity distribution determined for the nuclear medicine image, with reference to a cutoff frequency table storing information on a relation between a parameter related to a radial intensity distribution of a spatial frequency of a nuclear medicine image and a Butterworth filter cutoff frequency to be applied to the nuclear medicine image concerned; and filtering the nuclear medicine image data by means of a Butterworth filter set with the cutoff frequency.

An image processing program according to another aspect of the invention is a program for performing image processing on nuclear medicine image data, and causes a computer to execute the steps of: inputting a plurality of nuclear medicine image data of an object taken from different angles; applying a two-dimensional Fourier transform to a nuclear medicine image chosen from the plurality of inputted nuclear medicine images to obtain a spatial frequency of the chosen nuclear medicine image; calculating a radial intensity distribution of the spatial frequency of the chosen nuclear medicine image; determining a cutoff frequency corresponding to a parameter related to the radial intensity distribution determined for the chosen nuclear medicine image, with reference to a cutoff frequency table storing information on a relation between a parameter related to a radial intensity distribution of a spatial frequency of a nuclear medicine image and a Butterworth filter cutoff frequency to be applied to the nuclear medicine image concerned; filtering the plurality of nuclear medicine image data by means of a Butterworth filter set with the cutoff frequency; and reconstructing the plurality of filtered nuclear medicine image data to generate a reconstruction image of the object.

The image processing program of the invention may use the maximum value of frequency components contained in the radial intensity distribution or may use the sum total of each frequency component in the radial intensity distribution, as the parameter of the radial intensity distribution. The "frequency component" is a power spectrum corresponding to the frequency.

An image processing method of the invention comprises the steps of: preparing a cutoff frequency table storing information on a relation between a parameter related to a radial intensity distribution of a spatial frequency of a nuclear medicine image and a Butterworth filter cutoff frequency to be applied to the nuclear medicine image concerned; inputting nuclear medicine image data; applying a two-dimensional Fourier transform to the inputted nuclear medicine image to obtain a spatial frequency of the nuclear medicine image; calculating a radial intensity distribution of the spatial frequency of the nuclear medicine image; determining a cutoff frequency corresponding to a parameter related to the radial intensity distribution determined for the nuclear medicine image, with reference to the cutoff frequency table; and filtering the nuclear medicine image data by means of a Butterworth filter set with the cutoff frequency.

An image processing apparatus of the invention comprises: a cutoff frequency table storing information on a relation between a parameter related to a radial intensity distribution of a spatial frequency of a nuclear medicine image and a Butterworth filter cutoff frequency to be applied to the nuclear medicine image concerned; a nuclear medicine image input unit for inputting nuclear medicine image data; a Fourier transform unit for applying a two-dimensional Fourier transform to the inputted nuclear medicine image to obtain a spatial frequency of the nuclear medicine image; a radial intensity distribution calculation unit for calculating a radial intensity distribution of the spatial frequency of the nuclear medicine image; a cutoff frequency setting unit for setting a cutoff frequency corresponding to a parameter related to the radial intensity distribution determined for the nuclear medicine image, with reference to the cutoff frequency table; and a Butterworth filter for filtering the nuclear medicine image data with the set cutoff frequency.

In the image processing apparatus of the invention, the cutoff frequency table may include different tables depending on the type of collimator or on an equation for the Butterworth filter to be used, and the cutoff frequency setting unit may set the cutoff frequency corresponding to the parameter related to the radial intensity distribution determined for the nuclear medicine image, with reference to a table corresponding to the type of collimator used for taking the nuclear medicine image or to an equation for the Butterworth filter to be used.

In the invention, a cutoff frequency table storing information on a relation between a parameter related to a radial intensity distribution of a spatial frequency of a nuclear medicine image and a Butterworth filter cutoff frequency to be applied to the nuclear medicine image concerned is prepared in advance, and a cutoff frequency corresponding to an inputted nuclear medicine image is read from the cutoff frequency table and set, so that an optimum cutoff frequency can be set automatically regardless of the experience and skill of an individual operator.

There are other aspects of the invention as described below. This disclosure of the invention therefore intends to provide part of the invention and does not intend to limit the scope of the invention described and claimed herein.

BRIEF DESCRIPTION OF THE DRAWINGS

Figure 1 shows a configuration of an image processing apparatus of an embodiment;
Figure 2A shows an example of projection image data;
Figure 2B shows a result of a two-dimensional Fourier transform applied to the projection image data;
Figure 3A illustrates a method of calculating a radial intensity distribution;
Figure 3B shows an example of a calculated radial intensity distribution;
Figure 4A shows an example where an intensity distribution of frequency components in only one direction is determined;
Figure 4B shows the distribution of frequency components in the one direction;
Figure 5 shows an example of data stored in a cutoff frequency table;
Figure 6 is a flowchart showing an operation of the image processing apparatus;
Figure 7 shows a configuration of a program of the embodiment;
Figure 8 is a plot of a relation between the optimum cutoff frequency and the maximum value of frequency components in a radial intensity distribution, for a case where a head was imaged;
Figure 9A shows a reconstruction image of a head generated with a cutoff frequency automatically determined by the image processing apparatus;
Figure 9B shows a reconstruction image of the head generated with a cutoff frequency determined by experts' visual evaluation;
Figure 10 is a plot of a relation between the optimum cutoff frequency and the sum total of frequency components in a radial intensity distribution, for a case where a head was imaged;
Figure 11 is a plot of a relation between the optimum cutoff frequency and the maximum value of frequency components in a radial intensity distribution, for a case where heart muscles were imaged; and
Figure 12 is a plot of a relation between the optimum cutoff frequency and the sum total of frequency components in a radial intensity distribution, for a case where heart muscles were imaged.

BEST MODE OF EMBODYING THE INVENTION

The following is a detailed description of the invention. It will be understood that the embodiment described below is only an example of the invention, and the invention can be varied in various aspects. Therefore, the specific configurations and functions disclosed below do not limit the claims.
Now, an image processing apparatus of an embodiment of the invention will be described with reference to the drawings.

*[Configuration of the image processing apparatus]

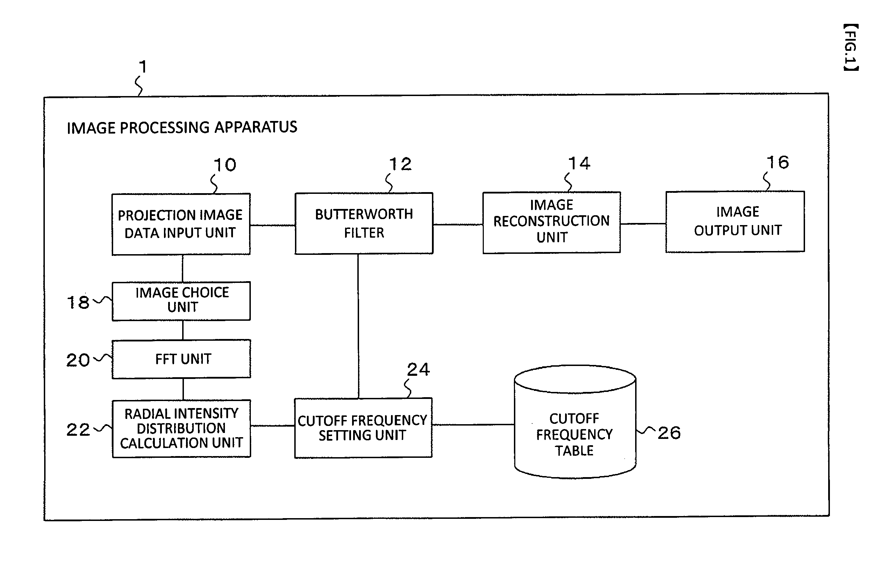

Figure 1 is a block diagram showing a configuration of an image processing apparatus 1 of the embodiment. The image processing apparatus 1 comprises: a projection image data input unit (hereinafter referred to as the "image input unit") 10 for inputting projection image (nuclear medicine image) data imaged by a PET or other imaging apparatus; a Butterworth filter 12 for filtering an image inputted to the image input unit 10; an image reconstruction unit 14 for reconstructing a filtered image to generate a three-dimensional image; and an image output unit 16 for outputting an image generated by the image reconstruction unit 14.
The image processing apparatus 1 comprises an image choice unit 18, a two-dimensional Fourier transform unit (hereinafter referred to as the "FFT unit") 20, a radial intensity distribution calculation unit 22, a cutoff frequency setting unit 24, and a cutoff frequency table 26, as a configuration for setting a cutoff frequency of the Butterworth filter 12.
The image choice unit 18 has a function to choose projection image data to be used for setting the cutoff frequency, from inputted projection image data. PET or the like uses, for example, 60 projection images of a subject taken every six degrees in order to generate a reconstruction image from projection images. The all projection image data may be used to set the cutoff frequency but, in the embodiment, the image choice unit 18 chooses a projection image to be used for setting the cutoff frequency in order to reduce the cutoff frequency setting process.
If a region to be subjected to image processing does not have much dependence on the imaging direction e.g. as a head does not, the image choice unit 18 may choose any projection image. If the region to be subjected to image processing is a heart for example, there is an influence of the liver or the like depending on the imaging direction, and thus the characteristics of a radial distribution function obtained from the projection images varies. In such a case, the image choice unit 18 chooses, for example, N images taken at 360 (degrees)/N intervals. The image choice unit 18 may also be configured to choose a projection image in accordance with the region. For example, if the target region is a heart, the image choice unit 18 chooses an image in which the liver and the heart are sufficiently separated from each other. Usually, an image obtained from the front or back of the patient can be used. If a 360-degree acquisition is performed, images obtained from the front and back of the patient may be summed up to improve the data sensitivity. This allows the image choice unit 18 to choose a projection image of the heart that is not much affected by the liver.

The FFT unit 20 applies a two-dimensional Fourier transform to a projection image chosen by the image choice unit 18 and obtains a two-dimensional spatial frequency of the projection image.

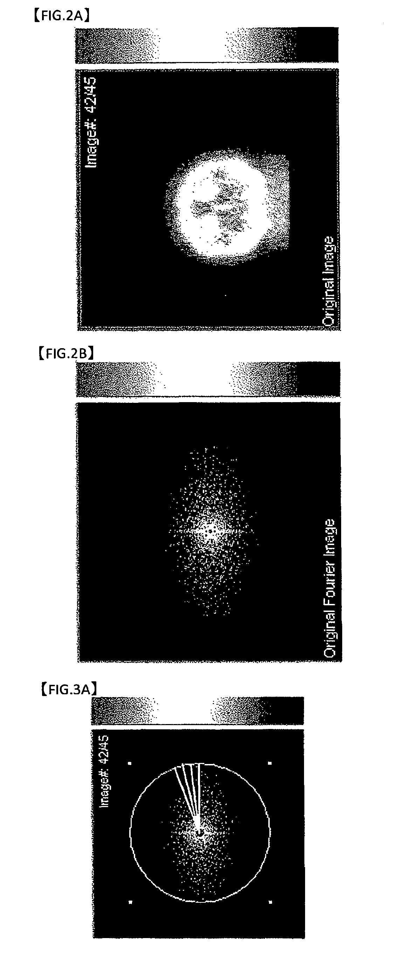

Figure 2A shows a chosen projection image, and Figure 2B shows a two-dimensional spatial frequency obtained by applying a two-dimensional Fourier transform to the projection image. The horizontal direction (X-axis) in Figure 2B represents the frequency component in the horizontal direction (X-axis) of the projection image shown in Figure 2A, and the vertical direction (Y-axis) in Figure 2B represents the frequency component in the vertical direction (Y-axis) of the projection image shown in Figure 2A.

Figures 2A and 2B presents the image in black and white, but the actual projection image and image representing the spatial frequency may show the signal intensity by color.

In a case where a plurality of projection images are chosen by the image choice unit 18,

(1) the two-dimensional Fourier transform may be applied after the plurality of projection images are combined together, or (2) the two-dimensional Fourier transform may be applied to each projection image and then, when the radial intensity distribution is determined, a process may be performed in which data determined from each chosen projection image are summed up.

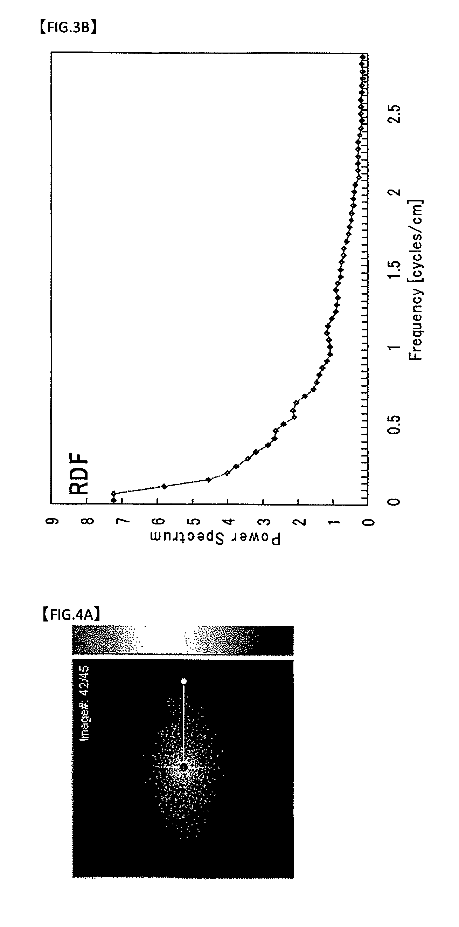

The radial intensity distribution calculation unit 22 determines a radial intensity distribution of a projection image transformed into frequency space by the two-dimensional Fourier transform. Figure 3A illustrates a method of calculating the radial intensity distribution, and Figure 3B shows an example of a calculated radial intensity distribution. As shown in Figure 3A, the radial intensity distribution calculation unit 22 determines the intensity of frequency components in a radial direction (360 directions, in this example) of the two-dimensional spatial frequency and adds up frequency components in each direction to determine the radial intensity distribution. In this way, the intensity of frequency components in each direction is added up and, as shown in Figure 3B, the radial intensity distribution indicating the intensity of each frequency component is determined. The radial intensity distribution calculation unit 22 may average frequency components in each direction after adding them up, by dividing by the number of data (360, in this example).

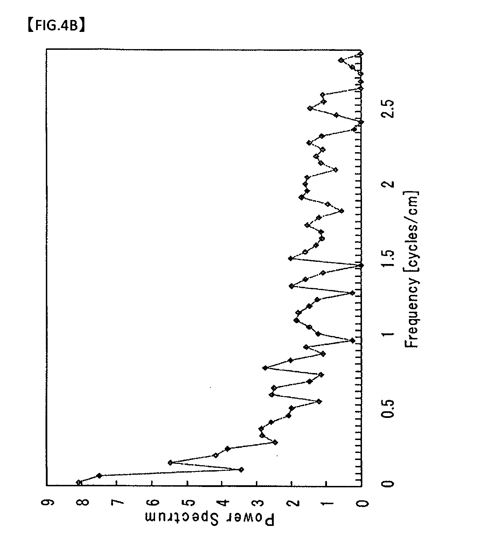

An intensity distribution of frequency components determined for only one direction (X-axis direction, in this example) as shown in Figure 4A would be significantly affected by noise and the like as shown in Figure 4B, but the determination of the radial intensity distribution cancels out the influence of noise and the like and provides a smooth frequency distribution as shown in Figure 3B.

The cutoff frequency setting unit 24 reads from the cutoff frequency table 26 a cutoff frequency corresponding to the maximum value of frequency components in a radial intensity distribution determined by the radial intensity distribution calculation unit 22, and sets the read cutoff frequency as a cutoff frequency of the Butterworth filter 12.

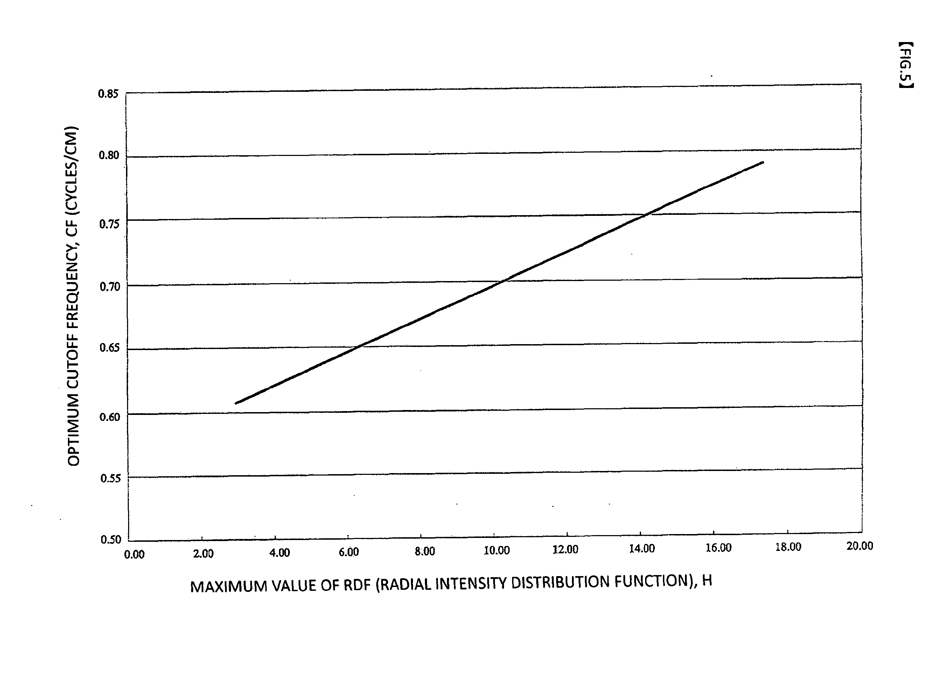

Figure 5 shows an example of data stored in the cutoff frequency table 26. The cutoff frequency table 26 stores information on a relation between the maximum value of frequency components in the radial intensity distribution and the cutoff frequency with which the Butterworth filter 12 should be set (the optimum cutoff frequency). The optimum cutoff frequency may be determined based on visual evaluation made by a plurality of experts, or may be determined by simulation. When it is determined by simulation, a method may be adopted in which, for example, a cutoff frequency that minimizes an error with respect to an ideal reconstruction image is determined as the optimum cutoff frequency.

While Figure 5 shows one example of data stored in the cutoff frequency table 26, different tables are prepared in the cutoff frequency table 26 depending on a region to be imaged, on the type of collimator to be applied to the imaging apparatus, or on an equation for the Butterworth filter to be used. The reason why tables are prepared for each type of collimator is because the resolution of a projection image differs depending on the type of collimator. The reason why different tables are prepared depending on an equation for the Butterworth filter to be used is because four equations for the Butterworth filter are at present known and the optimum cutoff frequency differs depending on the equation for the Butterworth filter to be used.

Now, a description will be made of a process of determining the optimum cutoff frequency by visual evaluation made by a plurality of experts. First, a plurality of reconstruction images are generated, with the cutoff frequency being varied, from projection image data obtained from one object, and experts choose therefrom an optimum reconstruction image. Subsequently, the maximum and minimum cutoff frequencies are removed from cutoff frequencies corresponding to the reconstruction images chosen by the experts, and the remaining cutoff frequencies are averaged to determine the optimum cutoff frequency. The removal of the maximum and minimum cutoff frequencies is to prevent the outliers from affecting the average value. The relation between the thus determined optimum cutoff frequency and the maximum frequency component in the radial intensity distribution of the spatial frequency of the projection image data concerned is determined, and this relation is stored in the cutoff frequency table.

[Operation of the image processing apparatus]

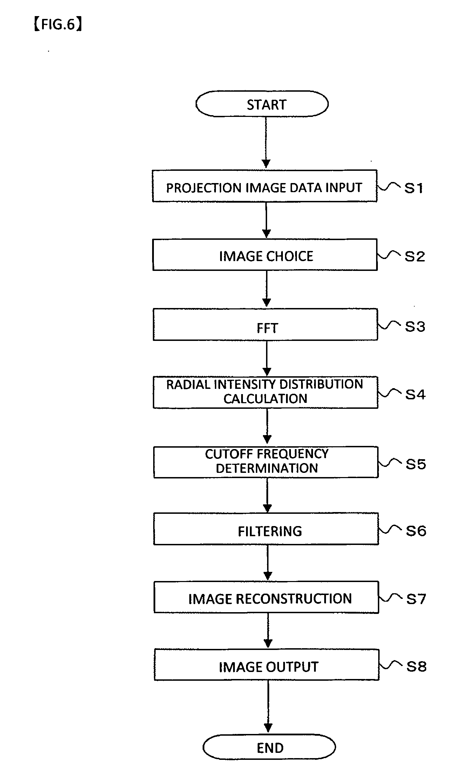

Figure 6 is a flowchart showing an operation of the image processing apparatus 1. First, the image processing apparatus 1 accepts an input of projection image data (S1). The image processing apparatus 1 here accepts inputs of a plurality of projection image data required to generate a reconstruction image. The image processing apparatus 1 then chooses, from the inputted projection image data, projection image data to be used for setting the cutoff frequency (S2). The image processing apparatus 1 applies a two-dimensional Fourier transform to the chosen projection image data to determine a two-dimensional spatial frequency (S3). Then, the image processing apparatus 1 calculates a radial intensity distribution of the two-dimensional spatial frequency (S4).

The image processing apparatus 1 reads from the cutoff frequency table 26 a cutoff frequency corresponding to the maximum value of frequency components in the radial intensity distribution, and sets the Butterworth filter 12 with the read cutoff frequency (S5). The image processing apparatus 1 filters the plurality of projection image data by means of the Butterworth filter 12 (S6). Subsequently, the image processing apparatus 1 reconstructs the filtered projection image data to generate a reconstruction image (S7), and outputs the generated reconstruction image (S8).

[Program]

While Figure 1 shows the configuration of the image processing apparatus 1 from a functional standpoint, the hardware of the image processing apparatus 1 is comprised of a computer comprising: a CPU; a ROM; a RAM; an input unit (e.g. a keyboard, a mouse, and an USB interface); an output unit (e.g. a monitor, a speaker, and an USB interface); a communications unit; and the like. The image processing apparatus 1 shown in Figure 1 is realized by the CPU of the computer reading and executing a program.

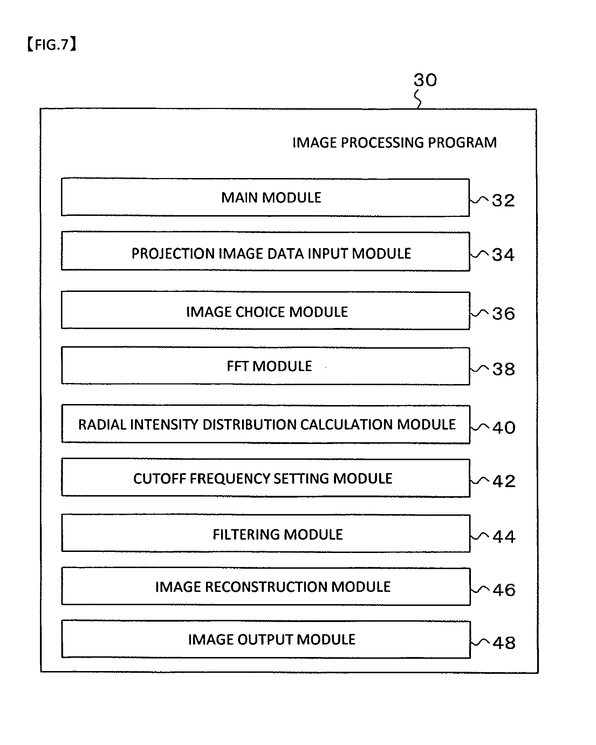

Figure 7 shows a configuration of an image processing program 30 of the embodiment. The image processing program 30 of the embodiment has: a main module 32; a projection image data input module (hereinafter referred to as the "image input module") 34; an image choice module 36; a two-dimensional Fourier transform module (hereinafter referred to as the "FFT module") 38; a radial intensity distribution calculation module 40; a cutoff frequency setting module 42; a filtering module 44; an image reconstruction module 46; and an image output module 48.

The main module 32 is a module for controlling the execution of: the image input module 34; the image choice module 36; the FFT module 38; the radial intensity distribution calculation module 40; the cutoff frequency setting module 42; the filtering module 44; the image reconstruction module 46; and the image output module 48.

When executed by the computer, the image input module 34 realizes the function of the image input unit 10 shown in Figure 1. This allows the computer to accept an input of projection image data to be subjected to image processing.

When executed by the computer, the image choice module 36 realizes the function of the image choice unit 18 shown in Figure 1. This allows the computer to choose, from the projection image data, projection image data to be used for setting the optimum cutoff frequency.

When executed by the computer, the FFT module 38 realizes the function of the FFT unit 20 shown in Figure 1. This allows the computer to apply a two-dimensional Fourier transform to projection image data to obtain a two-dimensional spatial frequency of the projection image data.

When executed by the computer, the radial intensity distribution calculation module 40 realizes the function of the radial intensity distribution calculation unit 22 shown in Figure 1. This allows the computer to determine a radial intensity distribution of a two-dimensional spatial frequency obtained by the two-dimensional Fourier transform.

When executed by the computer, the cutoff frequency setting module 42 realizes the function of the cutoff frequency setting unit 24 shown in Figure 1. This allows the computer to read from the cutoff frequency table 26 a cutoff frequency associated with the maximum value of frequency components in a radial intensity distribution, and to set the Butterworth filter 12 with the read cutoff frequency.

When executed by the computer, the filtering module 44 filters inputted projection image data by means of the Butterworth filter 12 with a cutoff frequency set by the cutoff frequency setting module 42.

When executed by the computer, the image reconstruction module 46 realizes the function of the image reconstruction unit 14 shown in Figure 1. The computer reconstructs filtered projection image data to generate a three-dimensional image.

When executed by the computer, the image output module 48 realizes the function of the image output unit 16 shown in Figure 1. The computer outputs a nuclear medicine image reconstructed by the image reconstruction module 46.

Recording media for recording the image processing program 30 of the embodiment include a hard disk, a flexible disk, a CD-ROM, a DVD, or other recording media, such as a ROM, or semiconductor memories. A recording medium in which the image processing program 30 is stored is inserted into a reader provided on the computer, and the image processing program 30 thereby becomes accessible by the computer, so that the image processing program 30 concerned allows the computer to operate as the image processing apparatus 1.

The image processing program 30 may be provided via a network as a computer data signal superimposed on a carrier wave. In this case, the image processing program 30 is received by a communications device provided on the computer and stored in a memory also provided on the computer. Then the image processing program 30 is executed by the computer.

Up to this point, there have been described the image processing apparatus 1, image processing method, and image processing program 30 for realizing them, of the embodiment.

In the embodiment, the cutoff frequency table 26 storing information on a relation between a parameter related to a radial intensity distribution of a spatial frequency of a nuclear medicine image and a cutoff frequency of the Butterworth filter 12 to be applied to the nuclear medicine image concerned is stored, and a cutoff frequency corresponding to an inputted nuclear medicine image is read from the cutoff frequency table 26 and set. This configuration allows an optimum cutoff frequency to be set automatically regardless of the experience and skill of an individual operator. Butterworth filtering with this cutoff frequency can provide nuclear medicine image data with reduced noise and can provide an appropriate diagnosis.

The cutoff frequency table 26 used in the embodiment is not susceptible to an individual difference between imaging apparatuses for nuclear medicine images and to a difference between radiopharmaceuticals. As a result, an appropriate cutoff frequency can be set with the one cutoff frequency table regardless of a difference between imaging apparatuses and between radiopharmaceuticals.

While in the above-described embodiment the maximum value of frequencies in the radial intensity distribution is used as the parameter related to the radial intensity distribution, the parameter related to the radial intensity distribution is not limited to the maximum value of frequency components. The sum total of frequency components may also be used as the parameter related to the radial intensity distribution.

Examples

Now, the invention will be described more specifically with working examples, the contents of which, however, does not limit the subject matter of the invention. In each working example, the order of the Butterworth filter was eight.

[Example 1] Case where a head was imaged: 1

In this working example, the optimum cutoff frequency was determined based on 10 experts' visual evaluation. The head of a subject was to be imaged. First, 10 reconstruction images were generated, with the cutoff frequency being varied, from projection image data obtained from the imaged target, and an optimum reconstruction image was chosen by each expert. In the working example, the projection image was taken with an SHR fan-beam collimator. The maximum and minimum cutoff frequencies were removed from 10 cutoff frequencies corresponding to the reconstruction images chosen by the experts, and the average value of the remaining eight cutoff frequencies was determined as the optimum cutoff frequency. The above-described procedure was performed on 42 cases of projection image data obtained by imaging 42 subjects, and the optimum cutoff frequency corresponding to each of the projection image data was determined.

Subsequently, data to be stored in the cutoff frequency table was generated based on the optimum cutoff frequency determined by the experts as described above. Specifically, the relation between the optimum cutoff frequency, CF, for the 42 cases of projection image data and the maximum frequency component, H, in the radial intensity distribution of the spatial frequency of the projection image data concerned was determined.

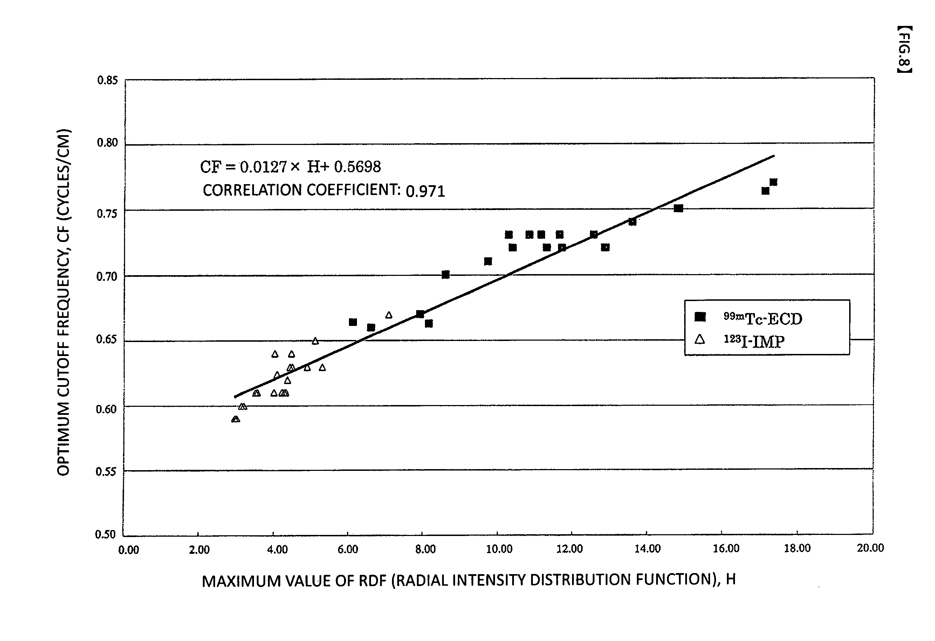

Figure 8 is a plot of the relation between the optimum cutoff frequency and the maximum value of frequency components in the radial intensity distribution, for the 42 cases. In Figure 8, the horizontal axis represents the maximum value of frequency components in the radial intensity distribution, and the vertical axis represents the optimum cutoff frequency (cycles/cm). In Figure 8, data represented by an open triangle is data for which $^{123}$I-IMP was used as a radiopharmaceutical, and data represented by a solid square is data for which $^{99m}$Tc-ECD was used as a radiopharmaceutical. As shown in Figure 8, a strong correlation with a correlation coefficient of 0.971 was found between the maximum value of frequency components in the radial intensity distribution and the optimum cutoff frequency, regardless of the type of radiopharmaceutical. Consequently, it turns out that, by storing information on this correlation in the cutoff frequency table, the optimum cutoff frequency can be determined based on the maximum value of frequency components in the radial intensity distribution. In this working example, a regression line was created by using the least squares method, a line $CF = 0.0127 \times H + 0.5698$ representing the correlation was determined, and information on this relational expression was stored in the cutoff frequency table 26.

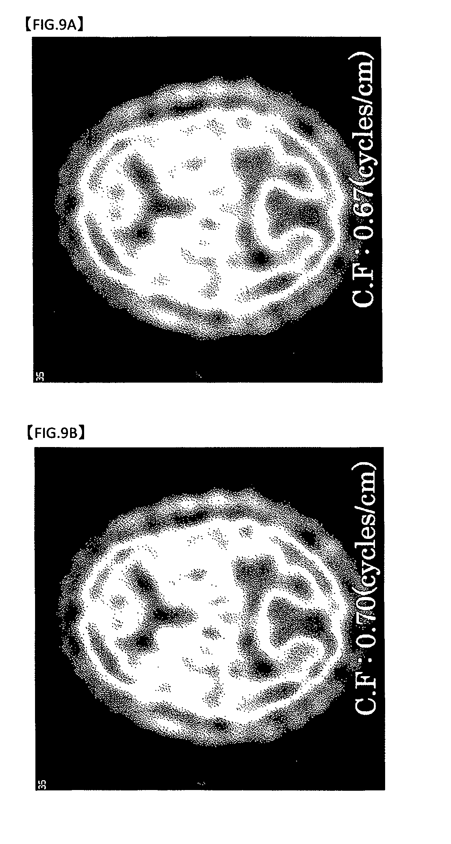

In the working example, the cutoff frequency table generated as described above was used to generate a reconstruction image. Figure 9A shows the reconstruction image generated by an image processing apparatus having the cutoff frequency table prepared through the above-described procedure. That is, the image processing apparatus determined a cutoff frequency to be applied to a projection image of a subject who was different from the subjects of the above-described 42 cases; a Butterworth filter set with the determined cutoff frequency was used to filter the projection image; and the reconstruction image was generated from the filtered projection image. The image processing apparatus determined the cutoff frequency to be 0.67.

Figure 9B shows a reconstruction image processed with a cutoff frequency which was determined by experts' visual evaluation to be applied to the projection image of the same subject. The experts' visual evaluation was done through the same procedure as the above-described generation of the cutoff frequency table. The experts determined the cutoff frequency to be 0.70.

As shown in Figures 9A and 9B, the reconstruction image generated by the image processing apparatus automatically determining the cutoff frequency was extremely similar to the reconstruction image determined by the experts' visual evaluation. In order to quantitatively evaluate the degree of approximation of both images, an error between both images was determined as below. The following expression (1) performs the error calculation by comparing a plurality of reconstructed slice images obtained with the cutoff frequency automatically determined and a plurality of reconstructed slice images obtained by the visual evaluation:

[Mathematical expression 1]

$$Error(\%) = \frac{\sum_{n=1}^{slice}\sum_{y=1}^{mtx}\sum_{x=1}^{mtx}\sqrt{\left(P_{auto}(n,x,y) - P_{visual}(n,x,y)\right)^2}}{\sum_{n=1}^{slice}\sum_{y=1}^{mtx}\sum_{x=1}^{mtx}P_{auto}(n,x,y)} \times 100 \quad (1)$$

where

$P_{auto}(n,x,y)$: Value of a pixel at the coordinates (x, y) in the automatically determined image of the nth slice;
$P_{visual}(n,x,y)$: Value of a pixel at the coordinates (x, y) in the visually evaluated image of the nth slice;
slice: Total number of slices; and
mtx: Matrix size of the reconstruction images.

The above-described numerical evaluation of the degree of approximation of the images resulted in an extremely small error of 1.6%.

[Example 2] Case where a head was imaged: 2

In this example, a table was generated which used the sum total of frequency components as the parameter related to the radial intensity distribution. The sum total of frequency components can be determined as the area of the region bounded by the graph of the radial intensity distribution and the horizontal and vertical axes, so in the example the area of the radial intensity distribution was used as the sum total of frequency components.

As in the case of the above-described Example 1, 10 experts determined the optimum cutoff frequency as well as the radial intensity distribution of projection image data, for the 42 cases of projection image data obtained by imaging the heads of the subjects.

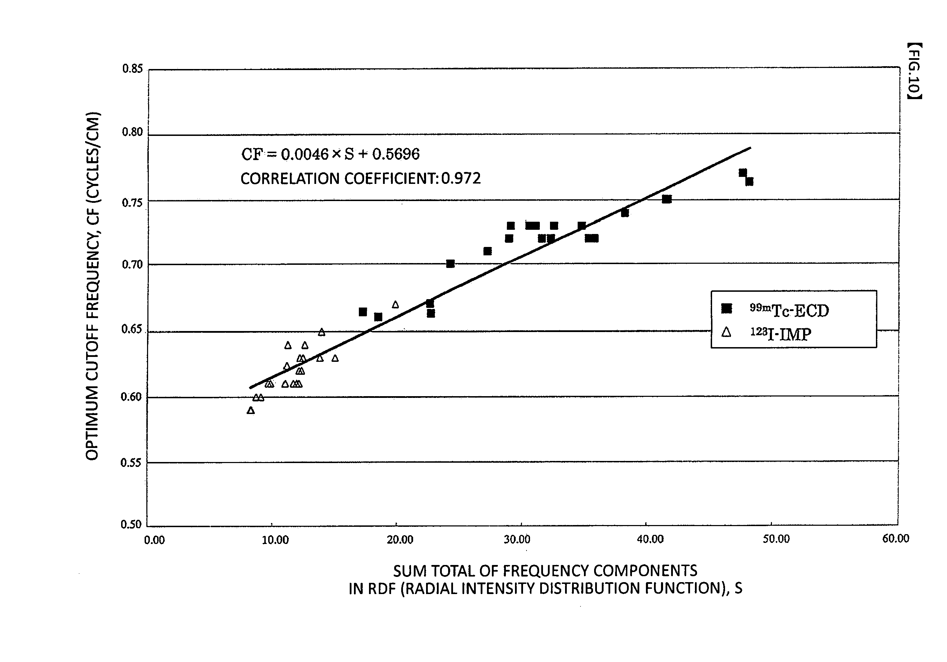

Figure 10 is a plot of the relation between the optimum cutoff frequency, CF, and the area, S, of the radial intensity distribution of the 42 cases. As shown in Figure 10, a strong correlation with a correlation coefficient of 0.972 was found between the area of the radial intensity distribution and the optimum cutoff frequency, regardless of the type of radiopharmaceutical. Consequently, it turns out that, by storing information on this correlation in the cutoff frequency table, the optimum cutoff frequency can be determined based on the sum total of frequency components in the radial intensity distribution. In this working example, a regression line was created by using the least squares method, a line CF = 0.0046 x S + 0.5696 representing the correlation was determined, and information on this relational expression was stored in the cutoff frequency table 26.

[Example 3] Case where heart muscles were imaged: 1

Also in this working example, the optimum cutoff frequency was determined based on 10 experts' visual evaluation. Heart muscles of a subject were imaged; 10 reconstruction images were generated, with the cutoff frequency being varied, from the obtained projection image data; and an optimum reconstruction image was chosen by experts. The maximum and minimum cutoff frequencies were removed from 10 cutoff frequencies corresponding to the reconstruction images chosen by the experts, and the average value of the remaining eight cutoff frequencies was determined as the optimum cutoff frequency.

In the working example, the above-described procedure was performed on 53 cases of projection image data obtained by imaging 53 subjects, and the optimum cutoff frequency corresponding to each of the projection image data was determined. In the working example, $^{99m}$Tc-tetrofosmin (35 cases) and $^{201}$TICI (18 cases) were used as radiopharmaceuticals. Projection images were acquired by using a GP collimator for 11 cases and projection images were taken by using an HR collimator for 24 cases, out of the 35 cases where $^{99m}$Tc-tetrofosmin was used. An HR collimator was used to take projection images for the 18 cases where $^{201}$TICI was used.

The radial intensity distribution of the projection image data was determined as below. Two pieces of projection image data taken from the front and back of a subject were chosen from the projection image data of the subject, and the chosen front and back projection image data were added together to generate an image. The reason why the images taken from the front and back of a subject were chosen is because those are not much affected by the liver. Subsequently, a square ROI (region of interest) was set in the heart muscle region; an image of the ROI concerned was extracted; and the extracted image of the ROI was linearly interpolated to generate an image of 128 x 128 pixels. A region where only the heart muscles showed up (region where the liver did not show up) was chosen as the ROI. The radial distribution function for the linearly interpolated image was then created, and the maximum value of frequency components was calculated by using the created radial distribution function.

Then, the relation between the optimum cutoff frequency for projection image data and the maximum frequency component in the radial intensity distribution of the spatial frequency of the projection image data concerned was determined for the 53 cases.

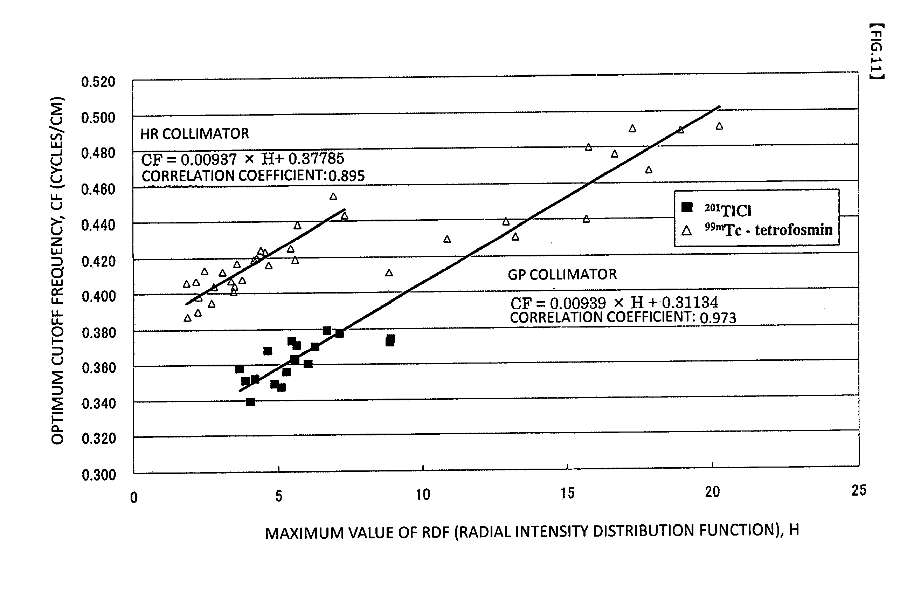

Figure 11 is a plot of the relation between the optimum cutoff frequency, CF, and the maximum value of frequency components, H, in the radial intensity distribution of the 53 cases. In Figure 11, data represented by an open triangle is data for which $^{99m}$Tc-tetrofosmin was used as a radiopharmaceutical, and data represented by a solid square is data for which $^{201}$TICI was used as a radiopharmaceutical. As shown in Figure 11, two different correlations were found depending on the type of collimator. As shown in Figure 11, projection images with a GP collimator showed a strong correlation with a correlation coefficient of 0.973 between the maximum value of frequency components in the radial intensity distribution and the optimum cutoff frequency, regardless of the type of radiopharmaceutical. Projection images with an HR collimator showed a strong correlation with a correlation coefficient of 0.895 between the maximum value of frequency components in the radial intensity distribution and the optimum cutoff frequency. Consequently, it turns out that, by storing information on these correlations in the cutoff frequency table, the optimum cutoff frequency can be determined based on the maximum value of frequency components in the radial intensity distribution.

From the data obtained by imaging with the GP collimator, a line CF = 0.00939 x H + 0.31134 representing the correlation was determined and information on this relational expression was stored in the cutoff frequency table 26 in association with information indicating the GP collimator. From the data obtained by imaging with the HR collimator, a line CF = 0.00937 x H + 0.37785 representing the correlation was determined and information on this relational expression was stored in the cutoff frequency table 26 in association with information indicating the HR collimator.

[Example 4] Case where heart muscles were imaged: 2

In this example, a table was generated which used the sum total of frequency components as the parameter related to the radial intensity distribution. The sum total of frequency components can be determined as the area of the region bounded by the graph of the radial intensity distribution and the horizontal and vertical axes, so in the example the area of the radial intensity distribution was used as the sum total of frequency components.

As in the case of the above-described Example 3, 10 experts determined the optimum cutoff frequency as well as the radial intensity distribution of projection image data, for the 53 cases of projection image data obtained by imaging heart muscles of the subjects.

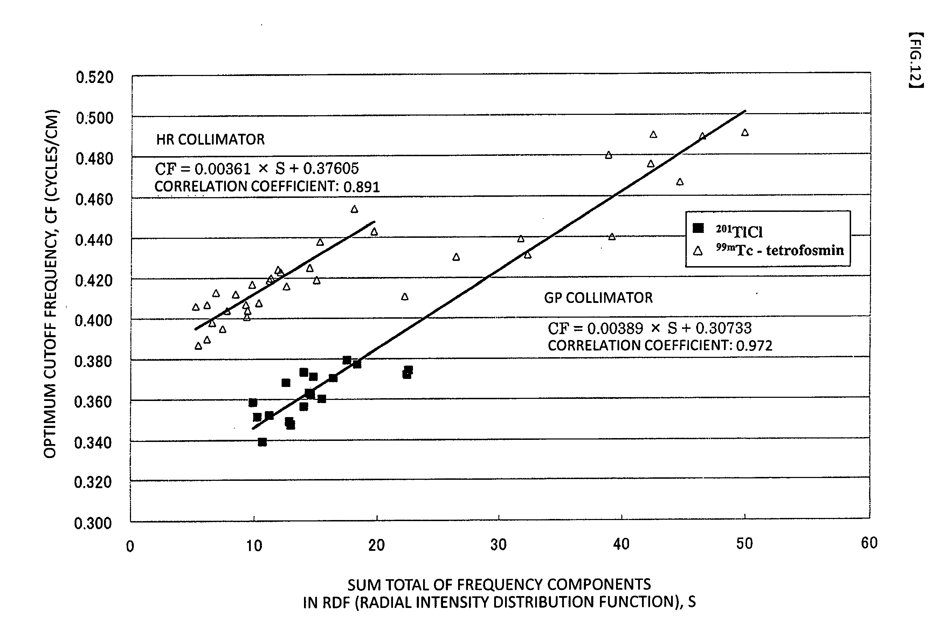

Figure 12 is a plot of the relation between the optimum cutoff frequency and the area of the radial intensity distribution, of the 53 cases, determined by the above-described procedure. In Figure 12, data represented by an open triangle is data for which $^{99m}$Tc-tetrofosmin was used as a radiopharmaceutical, and data represented by a solid square is data for which $^{201}$TICI was used as a radiopharmaceutical. As shown in Figure 12, two different correlations were found depending on the type of collimator. Projection images with a GP collimator showed a strong correlation with a correlation coefficient of 0.972 between the sum total of the radial intensity distribution and the optimum cutoff frequency, regardless of the type of radiopharmaceutical. Projection images with an HR collimator showed a strong correlation with a correlation coefficient of 0.891 between the sum total of the radial intensity distribution and the optimum cutoff frequency. Consequently, it turns out that, by storing information on these correlations in the cutoff frequency table, the optimum cutoff frequency can be determined based on the sum total of frequency components in the radial intensity distribution.

From the data obtained by imaging with the HR collimator, a line CF = 0.00361 x S + 0.37605 representing the correlation was determined and information on this relational expression was stored in the cutoff frequency table 26 in association with information indicating the HR collimator. From the data obtained by imaging with the GP collimator, a line CF = 0.00389 x S + 0.30733 representing the correlation was determined and information on this relational expression was stored in the cutoff frequency table 26 in association with information indicating the GP collimator.

While there has been described what is at present considered to be a preferred embodiment of the invention, it will be understood that various modifications and variations may be made thereto, and it is intended that appended claims cover all such modifications and variations as fall within the true spirit and scope of the invention.

Industrial applicability

The invention has an advantage of being able to automatically set an optimum cutoff frequency for a filter to be used for image processing of a nuclear medicine image regardless of the experience and skill of an individual operator, and is useful for PET, SPECT, and the like.

**Claims**

1.  A program for performing image processing on nuclear medicine image data, the image processing program causing a computer to execute the steps of:

    inputting nuclear medicine image data;
    applying a two-dimensional Fourier transform to the inputted nuclear medicine image to obtain a spatial frequency of the nuclear medicine image;
    calculating a radial intensity distribution of the spatial frequency of the nuclear medicine image;
    determining a cutoff frequency corresponding to a parameter related to the radial intensity distribution determined for the nuclear medicine image, with reference to a cutoff frequency table storing information on a relation between a parameter related to a radial intensity distribution of a spatial frequency of a nuclear medicine image and a Butterworth filter cutoff frequency to be applied to the nuclear medicine image concerned; and
    filtering the nuclear medicine image data by means of a Butterworth filter set with the cutoff frequency.

2.  A program for performing image processing on nuclear medicine image data, the image processing program causing a computer to execute the steps of:

    inputting a plurality of nuclear medicine image data of an object taken from different angles;
    applying a two-dimensional Fourier transform to a nuclear medicine image chosen from the plurality of inputted nuclear medicine images to obtain a spatial frequency of the chosen nuclear medicine image;
    calculating a radial intensity distribution of the spatial frequency of the chosen nuclear medicine image;
    determining a cutoff frequency corresponding to a parameter related to the radial intensity distribution determined for the chosen nuclear medicine image, with reference to a cutoff frequency table storing information on a relation between a parameter related to a radial intensity distribution of a spatial frequency of a nuclear medicine image and a Butterworth filter cutoff frequency to be applied to the nuclear medicine image concerned;
    filtering the plurality of nuclear medicine image data by means of a Butterworth filter set with the cutoff frequency; and
    reconstructing the plurality of filtered nuclear medicine image data to generate a reconstruction image of the object.

3.  The image processing program according to claim 1 or 2, wherein the maximum value of frequency components contained in the radial intensity distribution is used as the parameter of the radial intensity distribution.

4.  The image processing program according to claim 1 or 2, wherein the sum total of frequency components in the radial intensity distribution is used as the parameter of the radial intensity distribution.

5.  An image processing method comprising the steps of:

    preparing a cutoff frequency table storing information on a relation between a parameter related to a radial intensity distribution of a spatial frequency of a nuclear medicine image and a Butterworth filter cutoff frequency to be applied to the nuclear medicine image concerned;

inputting nuclear medicine image data;

applying a two-dimensional Fourier transform to the inputted nuclear medicine image to obtain a spatial frequency of the nuclear medicine image;

calculating a radial intensity distribution of the spatial frequency of the nuclear medicine image;

determining a cutoff frequency corresponding to a parameter related to the radial intensity distribution determined for the nuclear medicine image, with reference to the cutoff frequency table; and

filtering the nuclear medicine image data by means of a Butterworth filter set with the cutoff frequency.

6. An image processing apparatus comprising:

a cutoff frequency table storing information on a relation between a parameter related to a radial intensity distribution of a spatial frequency of a nuclear medicine image and a Butterworth filter cutoff frequency to be applied to the nuclear medicine image concerned;

a nuclear medicine image input unit for inputting nuclear medicine image data;

a Fourier transform unit for applying a two-dimensional Fourier transform to the inputted nuclear medicine image to obtain a spatial frequency of the nuclear medicine image;

a radial intensity distribution calculation unit for calculating a radial intensity distribution of the spatial frequency of the nuclear medicine image;

a cutoff frequency setting unit for setting a cutoff frequency corresponding to a parameter related to the radial intensity distribution determined for the nuclear medicine image, with reference to the cutoff frequency table; and

a Butterworth filter for filtering the nuclear medicine image data with the set cutoff frequency.

7. The image processing apparatus according to claim 6,

wherein the cutoff frequency table includes different tables depending on the type of collimator or on an equation for the Butterworth filter to be used, and

wherein the cutoff frequency setting unit sets the cutoff frequency corresponding to the parameter related to the radial intensity distribution determined for the nuclear medicine image, with reference to a table corresponding to the type of collimator used for taking the nuclear medicine image or to an equation for the Butterworth filter to be used.

【FIG.1】

【FIG.2A】

【FIG.2B】

【FIG.3A】

【FIG.3B】

【FIG.4A】

【FIG.4B】

【FIG.5】

【FIG.6】

```
                    ┌──────────────┐
                    │    START     │
                    └──────┬───────┘
                           │
                           ▼
            ┌──────────────────────────────┐
            │  PROJECTION IMAGE DATA INPUT  │∿ S1
            └──────────────┬───────────────┘
                           │
                           ▼
            ┌──────────────────────────────┐
            │         IMAGE CHOICE          │∿ S2
            └──────────────┬───────────────┘
                           │
                           ▼
            ┌──────────────────────────────┐
            │             FFT               │∿ S3
            └──────────────┬───────────────┘
                           │
                           ▼
            ┌──────────────────────────────┐
            │  RADIAL INTENSITY DISTRIBUTION │∿ S4
            │          CALCULATION          │
            └──────────────┬───────────────┘
                           │
                           ▼
            ┌──────────────────────────────┐
            │      CUTOFF FREQUENCY         │∿ S5
            │       DETERMINATION           │
            └──────────────┬───────────────┘
                           │
                           ▼
            ┌──────────────────────────────┐
            │           FILTERING           │∿ S6
            └──────────────┬───────────────┘
                           │
                           ▼
            ┌──────────────────────────────┐
            │     IMAGE RECONSTRUCTION      │∿ S7
            └──────────────┬───────────────┘
                           │
                           ▼
            ┌──────────────────────────────┐
            │         IMAGE OUTPUT          │∿ S8
            └──────────────┬───────────────┘
                           │
                           ▼
                    ┌──────────────┐
                    │     END      │
                    └──────────────┘
```

【FIG.7】

30

## IMAGE PROCESSING PROGRAM

| MAIN MODULE | ⟿32 |

| PROJECTION IMAGE DATA INPUT MODULE | ⟿34 |

| IMAGE CHOICE MODULE | ⟿36 |

| FFT MODULE | ⟿38 |

| RADIAL INTENSITY DISTRIBUTION CALCULATION MODULE | ⟿40 |

| CUTOFF FREQUENCY SETTING MODULE | ⟿42 |

| FILTERING MODULE | ⟿44 |

| IMAGE RECONSTRUCTION MODULE | ⟿46 |

| IMAGE OUTPUT MODULE | ⟿48 |

【FIG.8】

【FIG.9A】

C.F・0.67(cycles/cm)

【FIG.9B】

C.F・0.70(cycles/cm)

[FIG.10]

EP 2 202 539 A1

21

CF = 0.0046 × S + 0.5696
CORRELATION COEFFICIENT: 0.972

OPTIMUM CUTOFF FREQUENCY, CF (CYCLES/CM)

SUM TOTAL OF FREQUENCY COMPONENTS
IN RDF (RADIAL INTENSITY DISTRIBUTION FUNCTION), S

■ 99mTc-ECD
△ 123I-IMP

【FIG.11】

【FIG.12】

**HR COLLIMATOR**

$CF = 0.00361 \times S + 0.37605$
CORRELATION COEFFICIENT: 0.891

**GP COLLIMATOR**

$CF = 0.00389 \times S + 0.30733$
CORRELATION COEFFICIENT: 0.972

Legend:
■ $^{201}$TlCl
△ $^{99m}$Tc - tetrofosmin

Y-axis: OPTIMUM CUTOFF FREQUENCY, CF (CYCLES/CM)
X-axis: SUM TOTAL OF FREQUENCY COMPONENTS IN RDF (RADIAL INTENSITY DISTRIBUTION FUNCTION), S

## INTERNATIONAL SEARCH REPORT

| International application No. |
| --- |
| PCT/JP2008/002131 |

A. CLASSIFICATION OF SUBJECT MATTER
*G01T1/161*(2006.01)i, *A61B6/03*(2006.01)i, *G06T1/00*(2006.01)i, *G06T5/00* (2006.01)i, *G06T5/20*(2006.01)i

According to International Patent Classification (IPC) or to both national classification and IPC

B. FIELDS SEARCHED

Minimum documentation searched (classification system followed by classification symbols)
G01T1/00-7/12, A61B6/00-6/14, G06T1/00, G06T5/00, G06T5/20

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII)

C. DOCUMENTS CONSIDERED TO BE RELEVANT

| Category* | Citation of document, with indication, where appropriate, of the relevant passages | Relevant to claim No. |
| --- | --- | --- |
| A | JP 2000-338251 A (Hitachi Medical Corp.), 08 December, 2000 (08.12.00), Full text; all drawings (Family: none) | 1-7 |
| A | Chietsugu KATO, "Shuhasu Kukan Butterworth Filter Oyobi Wiener Filter o Mochiita Kaku Igaku Gazo Shori ni Kansuru Kisoteki Oyobi Rinshoteki Kento", Japanese Journal of Nuclear Medicine, 20 June, 1994 (20.06.94), Vol.31, No.6, pages 565 to 579 | 1-7 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 September, 2008 (08.09.08) | 22 September, 2008 (22.09.08) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007267859 A


**Non-patent literature cited in the description**

- *Japanese Journal of Nuclear Medicine Technology,* 2004, vol. 24 (2), 95-118